# EUROPEAN PATENT APPLICATION

(11) **EP 4 629 259 A1**
(43) Date of publication of application: **08.10.2025**
(21) Application number: 24741507.8
(22) Date of filing: 09.01.2024
(51) Int. Cl.: G16H 20/17, A61M 5/142, A61M 5/168

(54) **COMPUTER PROGRAM, DRUG LIBRARY EDITING DEVICE, DRUG LIBRARY EDITING SYSTEM, AND DRUG LIBRARY EDITING METHOD**

(30) Priority: 10.01.2023 JP 2023001825
(71) Applicant: Terumo Kabushiki Kaisha, Tokyo 151-0072 (JP)
(72) Inventor: HARA, Yuki, Ashigarakami-gun, Kanagawa 259-0151 (JP); ASAMA, Koichiro, Ashigarakami-gun, Kanagawa 259-0151 (JP); KOYAMA, Takahiro, Ashigarakami-gun, Kanagawa 259-0151 (JP)
(74) Representative: KIPA AB
(86) International application number: PCT/JP2024/000147
(87) International publication number: WO 2024/150734

(57) **Abstract**

Provided are a computer program, a drug library editing device, a drug library editing system, and a drug library editing method that allow a person in charge of editing a drug library to easily check a drug library displayed on a medical pump.

The computer program causes a computer to execute: acquiring a drug library including administration information for each drug; accepting an editing operation of a display format of the drug library displayed on a display unit of a medical pump based on the acquired drug library; and displaying preview information of a drug library displayed on the display unit in a display format corresponding to the accepted editing operation.

## Description

### Technical Field

The present invention relates to a computer program, a drug library editing device, a drug library editing system, and a drug library editing method.

### Background Art

A medical pump such as an infusion pump or a syringe pump is a medical device for administering various combinations of drugs necessary for treatment through an indwelling needle punctured into a vein and an infusion line in order to maintain a body fluid of a patient during treatment or medical treatment in a normal state. The medical pump can deliver a drug to a patient in a state in which management of the amount and time of drug administration is strictly required while controlling the flow rate of the drug.

Patent Literature 1 discloses a medical pump that stores a drug library in which information such as a flow rate of a drug and a limit value of the flow rate is defined for each type of drug, and can prevent erroneous administration due to a human error such as an order of magnitude or numerical difference when a medical worker such as a nurse sets the flow rate. Such a drug library can be edited using a predetermined application (for example, a library manager), and the edited drug library can be written in the medical pump.

### Citation List

### Patent Literature

Patent Literature 1: JP 2015-91280 A

### Summary of Invention

### Technical Problem

In general, editing work of the drug library is performed by an expert such as a pharmacist, and writing work of the edited drug library to the medical pump is performed by a system engineer or the like, and the person in charge of each work is different. Therefore, the editor of the drug library does not know how the drug library is displayed on the display screen of the medical pump. In addition, how the drug library is displayed on the display screen of the medical pump needs to be confirmed by the medical pump in which the drug library is actually written, and the confirmation work takes time and effort.

The present invention has been made in view of such circumstances, and an object of the present invention is to provide a computer program, a drug library editing device, a drug library editing system, and a drug library editing method that allow a person in charge of editing a drug library to easily check a drug library displayed on a medical pump.

### Solution to Problem

(1) A computer program according to the present invention causes a computer to execute: acquiring a drug library including administration information for each drug; accepting an editing operation of a display format of the drug library displayed on a display unit of a medical pump based on the acquired drug library; and displaying preview information of a drug library displayed on the display unit in a display format corresponding to the accepted editing operation.
   According to embodiments of the present invention, the following are provided.
(2) In the computer program according to (1), the editing operation includes a display order of drugs included in the drug library, and the computer program causes a computer to execute displaying preview information of a list of drugs in a display order according to the accepted editing operation.
(3) In the computer program according to (1) or (2), the editing operation includes setting of page delimiter when displaying a list of drugs included in the drug library, and the computer program causes a computer to execute displaying preview information of a list of drugs in page delimiter corresponding to the accepted editing operation.
(4) In the computer program according to any one of (1) to (3), the editing operation includes a display order of at least one item of a drug name, a color of a drug syringe, and a drug group, and the computer program causes a computer to execute displaying preview information of a list of drugs in a display order of items according to the accepted editing operation.
(5) In the computer program according to (4), the editing operation includes setting of page delimiter when displaying a list of drugs in a display order of the items, and the computer program causes a computer to execute displaying preview information of a list of drugs in page delimiter corresponding to the accepted editing operation.
(6) In the computer program of any one of (1) to (5), the preview information includes administration information of a selected drug displayed on the display unit in a case where the drug included in the drug library displayed on the display unit is selected.
(7) The computer program according to any one of (1) to (6) causes a computer to execute displaying: an editing operation accepting screen that accepts the editing operation; a page delimiter screen that includes page delimiter information of a drug library corresponding to the accepted editing operation; and a preview screen that includes preview information of a drug library displayed on the display unit in a display format corresponding to the accepted editing operation.
(8) A drug library editing device according to the present invention includes a control unit, in which the control unit executes: acquiring a drug library including administration information for each drug; accepting an editing operation of a display format of the drug library displayed on a display unit of a medical pump based on the acquired drug library; and displaying preview information of a drug library displayed on the display unit in a display format corresponding to the accepted editing operation.
(9) A drug library editing system according to the present invention includes: the drug library editing device; and a medical pump in which a drug library edited by the drug library editing device is written.
(10) A drug library editing method according to the present invention includes: acquiring a drug library including administration information for each drug; accepting an editing operation of a display format of the drug library displayed on a display unit of a medical pump based on the acquired drug library; and displaying preview information of a drug library displayed on the display unit in a display format corresponding to the accepted editing operation.

### Advantageous Effects of Invention

According to the present invention, a person in charge of editing a drug library can easily check the drug library displayed on the medical pump.

### Brief Description of Drawings

Fig. 1 is a diagram illustrating an example of a configuration of a drug library editing system of the present embodiment.
Fig. 2 is a diagram illustrating an example of a configuration of a syringe pump.
Fig. 3 is a diagram illustrating an example of an initial screen of a drug library editing screen.
Fig. 4 is a diagram illustrating an example of a color code.
Fig. 5 is a diagram illustrating an example of a drug library editing screen in a case where a person in charge of editing work performs editing of rearranging drug names in ascending order.
Fig. 6 is a diagram illustrating an example of a drug library editing screen in a case where a person in charge of editing work performs editing of rearranging drug names in descending order.
Fig. 7 is a diagram illustrating an example of a drug library editing screen in a case where a person in charge of editing work performs editing of rearranging colors in descending order.
Fig. 8 is a diagram illustrating an example of a drug library editing screen in a case where a person in charge of editing work sorts colors in descending order and adds a delimiter for each item.
Fig. 9 is a diagram illustrating an example of a drug library editing screen in a case where a person in charge of editing work performs editing of rearranging groups in descending order.
Fig. 10 is a diagram illustrating an example of a drug library editing screen in a case where a person in charge of editing work sorts groups in descending order and adds a delimiter for each item.
Fig. 11A is a diagram illustrating an example of a drug library displayed on a display unit of a syringe pump.
Fig. 11B is a diagram illustrating an example of a drug library displayed on the display unit of the syringe pump.
Fig. 12 is a diagram illustrating an example of a processing procedure by the drug library editing device.

### Description of Embodiments

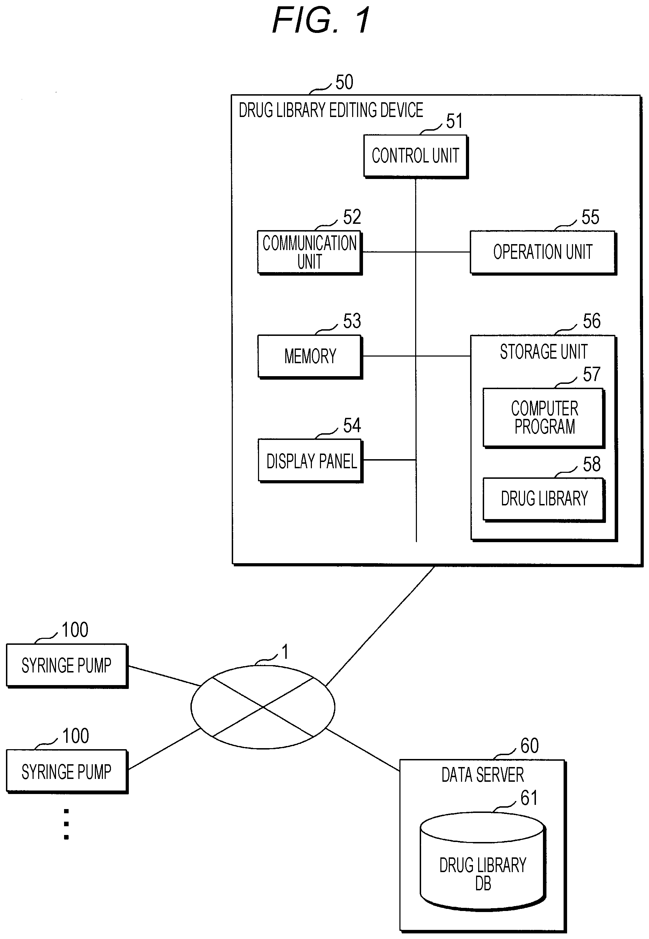

An embodiment of the present invention will be described below. Fig. 1 is a diagram illustrating an example of a configuration of a drug library editing system according to the present embodiment. The drug library editing system of the present embodiment includes a drug library editing device 50 and a syringe pump 100 as a plurality of medical pumps. In the present specification, a configuration using a syringe pump that injects a relatively small amount of drug as the medical pump will be described, but the medical pump is not limited to the syringe pump, and may be an infusion pump that injects a relatively large amount of drug. The drug library editing device 50 is connected to the syringe pump 100 via a communication network 1 such as a wired LAN or a wireless LAN. The drug library editing system may include a data server 60 having a drug library DB 61. The drug library editing device 50 is connected to the data server 60 via the communication network 1. In addition, each syringe pump 100 is connected to an in-hospital IT system (not illustrated) via the communication network 1 or another in-hospital communication system. The in-hospital IT system includes, for example, a hospital information system such as an electronic medical record, an assistance system that assists examination and treatment, a surgery/anesthesiology system, and the like.

The drug library editing device 50 includes a control unit 51 that controls the entire device, a communication unit 52, a memory 53, a display panel 54, an operation unit 55, and a storage unit 56. The drug library editing device 50 can be configured by, for example, a desktop or notebook personal computer, but may be configured by a tablet terminal.

The control unit 51 may be configured by incorporating a required number of chips, central processing units (CPUs), micro-processing units (MPUs), graphics processing units (GPUs), and the like. The control unit 51 may be configured by combining digital signal processors (DSPs), field-programmable gate arrays (FPGAs), and the like.

The communication unit 52 includes a communication module and has a function of communicating with the syringe pump 100 and the data server 60 via the communication network 1.

The display panel 54 can include a liquid crystal panel, an organic electro luminescence (EL) display, or the like.

The operation unit 55 includes, for example, a keyboard, a mouse, and the like, and can operate an icon, and the like, displayed on the display panel 54, move and operate a cursor, input characters, and the like. The operation unit 55 may include a touch panel.

The storage unit 56 can be configured by, for example, a hard disk, a semiconductor memory, or the like, and stores a computer program 57 (program product), a drug library 58, and necessary information (for example, a processing result by the drug library editing device 50, and the like).

The computer program 57 is an application program (library manager) for editing the drug library, and may be downloaded from an external device via the communication unit 52 and stored in the storage unit 56. The computer program 57 recorded in a recording medium (for example, an optically readable disk storage medium such as a CD-ROM) may be read by a recording medium read unit, and stored in the storage unit 56. The computer program 57 can be loaded so as to be executed on a single computer or on a plurality of computers which are located at one site or distributed across a plurality of sites and interconnected by a communication network.

The memory 53 may be configured by a semiconductor memory, such as a static random access memory (SRAM), a dynamic random access memory (DRAM), or a flash memory. The control unit 51 is capable of executing the computer program 57 deployed in the memory 53. The control unit 51 may execute processing defined by the computer program 57. That is, the processing executed by the control unit 51 corresponds to processing executed in accordance with the computer program 57.

The drug library 58 is obtained by acquiring a required drug library from the drug library DB 61 storing a plurality of drug libraries via the communication unit 52 and storing the drug library in the storage unit 56. The drug library 58 includes both the drug library acquired from the drug library DB 61 and the drug library edited by the drug library editing device 50.

The drug library 58 includes administration information for each drug. Specifically, the administration information includes, for example, setting information such as a reference flow rate, upper limit/lower limit values of the flow rate, a drug code, and a drug color for each of thousands of kinds of drugs. A collection of a plurality of pieces of setting information for one drug is also referred to as a "drug profile". By using the drug library, the medical worker does not need to perform complicated administration setting each time, and selection of a drug and setting of a drug are facilitated. For example, erroneous administration due to a setting error such as a flow rate (mL/h) and a dose (mL) can be prevented.

Since the drug library includes a relatively large number of types of drugs, it is difficult for a medical worker who uses a medical pump to select a required drug, and there is a possibility that selection errors occur. Therefore, as one method of classifying a large number of drugs included in a drug library, for example, a profile for narrowing down the drug library is determined for each surgery (Ope), intensive care unit (ICU), outpatient, and ward.

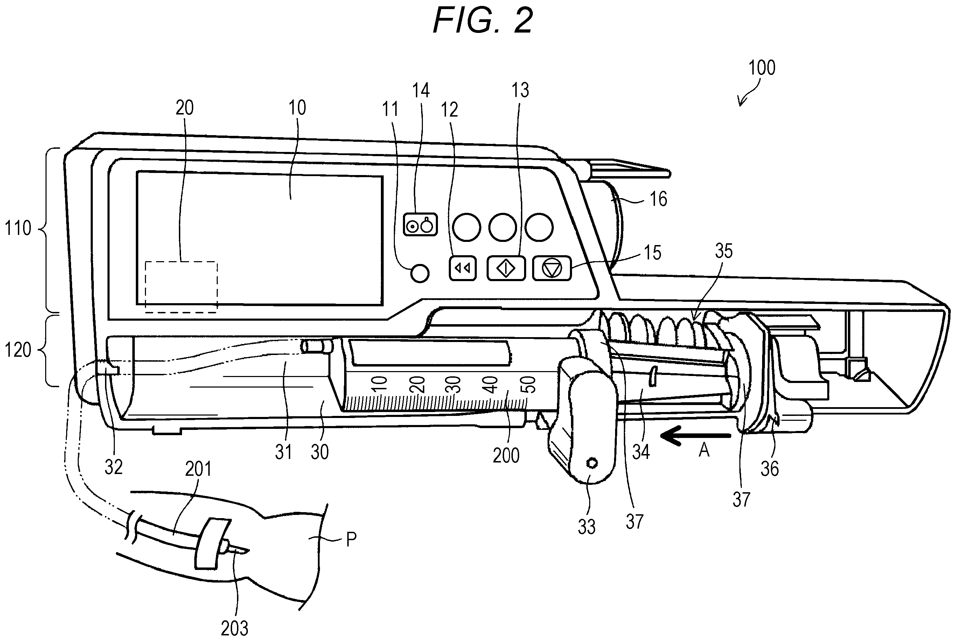

Fig. 2 is a diagram illustrating an example of a configuration of the syringe pump 100. The main body of the syringe pump 100 has a body upper portion 110 provided with a display unit 10 and a body lower portion 120 accommodating a syringe 200. The body lower portion 120 includes a placement portion 30 on which the syringe 200 is placed, a housing portion 31 which is a recess having a semicircular cross section for housing the syringe 200, a fixing portion 32 which is provided at an end portion of the housing portion 31 and removably sandwiches the tube 201, a clamp 33 for fixing the syringe 200 placed on the placement portion 30, a syringe plunger driving unit 35 which includes a slider 36 for fixing a plunger flange 37 provided on the syringe 200 and a motor (not illustrated) and pushes a syringe plunger 34 provided in the syringe 200, and the like.

A controller 20 is provided in the body upper portion 110. The controller 20 controls the operation of the syringe pump 100. The controller 20 includes a microcomputer (microprocessor), a read only memory (ROM), a random access memory (RAM), a non-volatile memory, a clock, and the like. The clock is used to acquire the current time, measure an elapsed time of a predetermined liquid feeding work, measure a reference time of speed control of liquid feeding, and the like.

The body upper portion 110 has an operation unit having various operation buttons and display lamps. The operation unit includes, for example, an operation indicator 11 that displays an operation state of the syringe pump 100, a fast-forward switch button 12, a start switch button 13, a power on/off button 14, a stop switch button 15, and the like. The operation unit may be provided with another operation button. At an end portion of the body upper portion 110, a setting dial 16 for setting a flow rate and a dose of the drug, a weight of the patient, and the like is provided.

The power on/off button 14 is used to turn on/off the power of the syringe pump 100. The fast-forward switch button 12 is used to fill the tube connected to the syringe 200 and the tip of an indwelling needle 203 with the drug. The start switch button 13 is used to start liquid feeding. The stop switch button 15 is used to temporarily stop liquid feeding in the middle.

As a method of setting the flow rate of liquid feeding or the like, there are various modes such as a mL/h mode, a µg/kg/min mode, a mg/kg/h mode, and a drug library mode. In the case of using the drug library mode, a previously registered drug or administration method is selected, and a dose or the like corresponding to the selected drug or administration method is set using the setting dial 16.

The syringe pump 100 continuously feeds the medicinal solution in the syringe at a set flow rate (mL/h). The controller 20 controls liquid feeding by controlling a motor rotation signal on the basis of setting information set for each drug. The syringe plunger driving unit 35 (motor) can feed liquid by pushing the syringe plunger 34 in the direction of reference sign A in Fig. 2 via the slider 36 or the like. As a result, the medical worker can inject the drug in the syringe 200 into a patient P through the tube 201 and the indwelling needle 203.

Next, a method for a person in charge of editing the drug library to edit the drug library by using the drug library editing device 50 will be described.

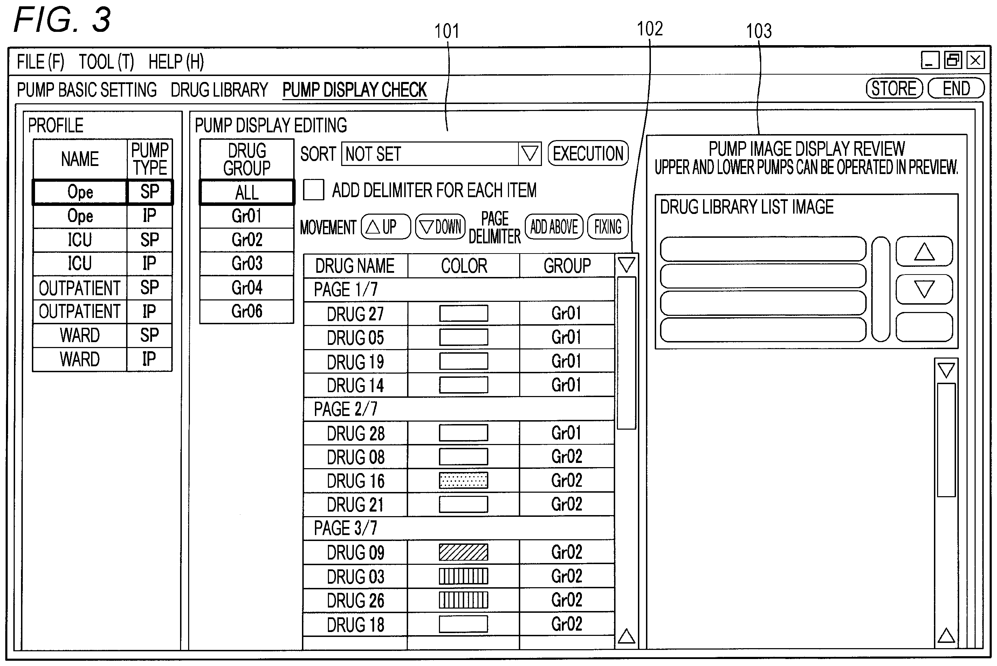

Fig. 3 is a diagram illustrating an example of an initial screen of a drug library editing screen. The drug library editing screen including the initial screen includes an editing operation accepting screen 101 for accepting an editing operation, a page delimiter screen 102 for displaying page delimiter information of the drug library corresponding to the accepted editing operation, and a preview screen 103 for displaying preview information of the drug library displayed on the display unit 10 of the syringe pump 100 in a display format corresponding to the accepted editing operation.

The drug library editing screen includes a profile display screen for selecting a profile and a drug group display screen for selecting a drug group. The person in charge of editing work can select a syringe pump (SP) or an infusion pump (IP) from among surgery (Ope), an intensive care unit (ICU), an outpatient, and a ward on the profile display screen. The person in charge of editing work can select whether to select all the drug groups or select individual groups Gr01 to Gr06 in the selected profile on the drug group display screen.

The drug groups are grouped according to the type, use, and the like of the drug. The drug groups are denoted by reference numerals such as Gr01 to Gr06 for convenience in the present specification, but can be actually classified into categories including anticancer agents, blood transfusion agents, nutrients, intravenous anesthetics, vasoactive agents, and the like. In the example of Fig. 3, drug libraries of all drug groups used in the syringe pump of surgery (Ope) are selected from among a large number of drugs.

The person in charge of editing work can perform "sort" setting, "add delimiter for each item" setting, and "page delimiter" setting on the editing operation accepting screen 101.

The person in charge of editing work can set any of a plurality of items (in the example of the drawing, three items of drug name, color, and group) and set the display order of the set items (in the example of the drawing, the ascending order or the descending order) in the "sort" setting. The person in charge of editing work can sort the information of the drug library for each drug name, color (color code), and group by the "sort" setting.

The display unit 10 of the syringe pump 100 is designed to display a predetermined number of drugs per screen (per page). The predetermined number is, for example, four. In this case, in a case where the "add delimiter for each item" setting is not made, four drugs are displayed in a list per screen on the display unit 10 of the syringe pump 100. On the other hand, when four drugs are displayed on one screen, it may be difficult to recognize the drugs. Therefore, the person in charge of editing work can set "add delimiter for each item" so that the number of drugs displayed on one screen can be freely set. In a case where the person in charge of editing work sets "add delimiter for each item", the "page delimiter" can be set at a required position, and the number of drugs displayed on one screen can be freely set.

On the page delimiter screen 102, three items of a drug name, a color, and a group are displayed in a list format for each drug. In a case where the person in charge of editing work makes a setting of "add delimiter for each item" on the editing operation accepting screen 101, the page delimiter can be set at a required position while moving the page delimiter up and down on the page delimiter screen 102. As a result, the person in charge of editing work can delimit the page for each item of the drug name, the color, or the group.

The preview screen 103 displays information of the drug library displayed on the display unit 10 of the syringe pump 100 without actually writing the edited drug library in the syringe pump 100. As a result, the person in charge of editing work can grasp how the edited information of the drug library is displayed on the display unit 10 of the syringe pump 100. The person in charge of editing work does not need to perform a separate work and check the drug library with the syringe pump 100 in which the drug library is actually written, and the person in charge of editing work can easily check the drug library displayed on the medical pump.

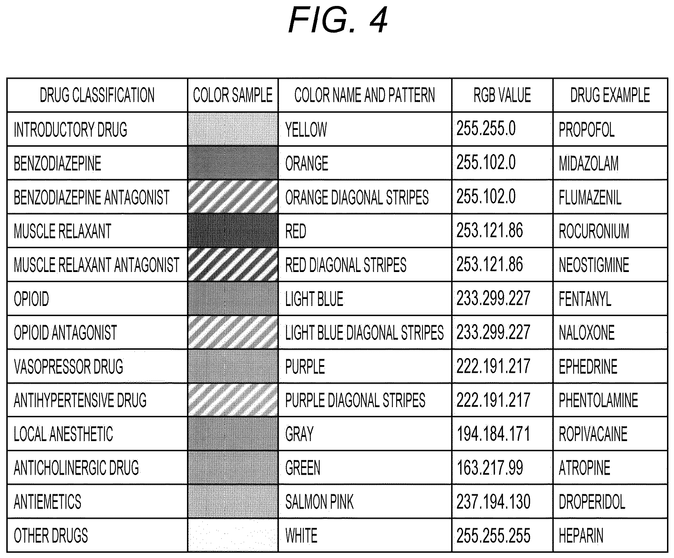

Fig. 4 is a diagram illustrating an example of a color code. The color code is a standard color code of a drug syringe label, and a color code formulated by the International Organization for Standardization (ISO) has been introduced also in Japan. As illustrated in Fig. 4, in the color code, a color name, a pattern (color sample), and an RGB value are determined for each drug classification. By attaching a color code label matching a drug in the syringe to the syringe, it is expected as one of measures to prevent erroneous drug such as erroneous administration.

Next, a specific editing operation will be described.

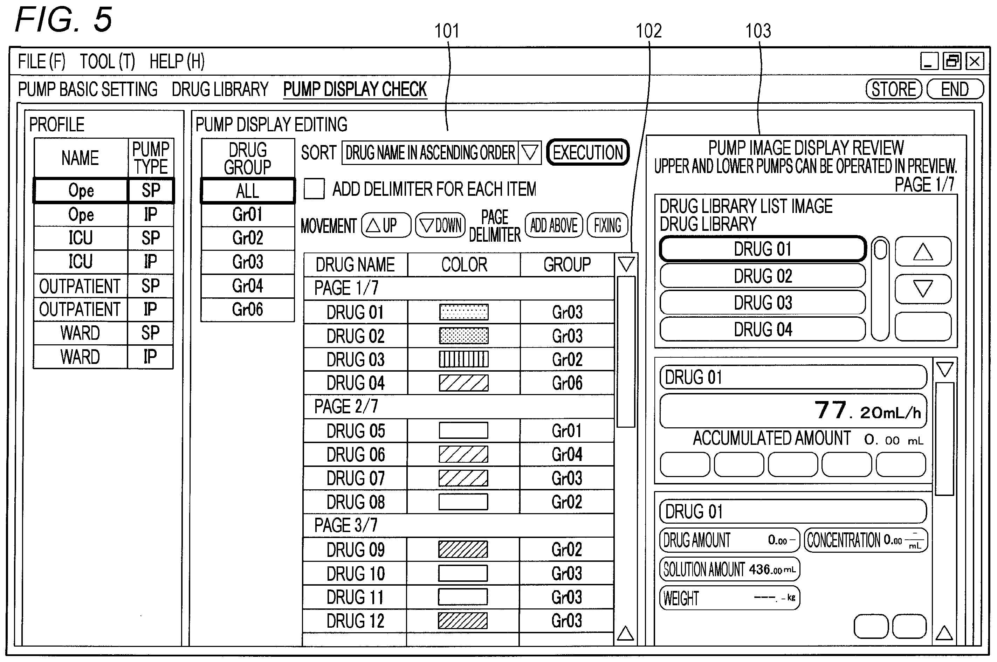

Fig. 5 is a diagram illustrating an example of a drug library editing screen in a case where the person in charge of editing work performs editing to sort drug names in ascending order. The control unit 51 displays all drugs in the drug library in the form of a list in ascending order of drug names on the page delimiter screen 102. When displayed in a list form, four drugs are displayed per page. Specifically, all the drugs in the drug library are delimited over 7 pages, the first page includes a drug 01, a drug 02, a drug 03, and a drug 04, the second page includes a drug 05, a drug 06, a drug 07, and a drug 08, and the third page includes a drug 09, a drug 10, a drug 11, and a drug 12. The same applies to the fourth and subsequent pages.

On the preview screen 103, four drugs 01, 02, 03, and 04 of the first page are displayed, and the second and subsequent pages can be similarly displayed by a scroll operation. When the person in charge of editing work selects the drug 01 among the four drugs on the first page, the control unit 51 can display administration information on the drug 01. In the example of Fig. 5, the flow rate (mL/h), the dose, and the like of the drug 01 are displayed. The preview screen 103 is an example and is not limited to the example of Fig. 5.

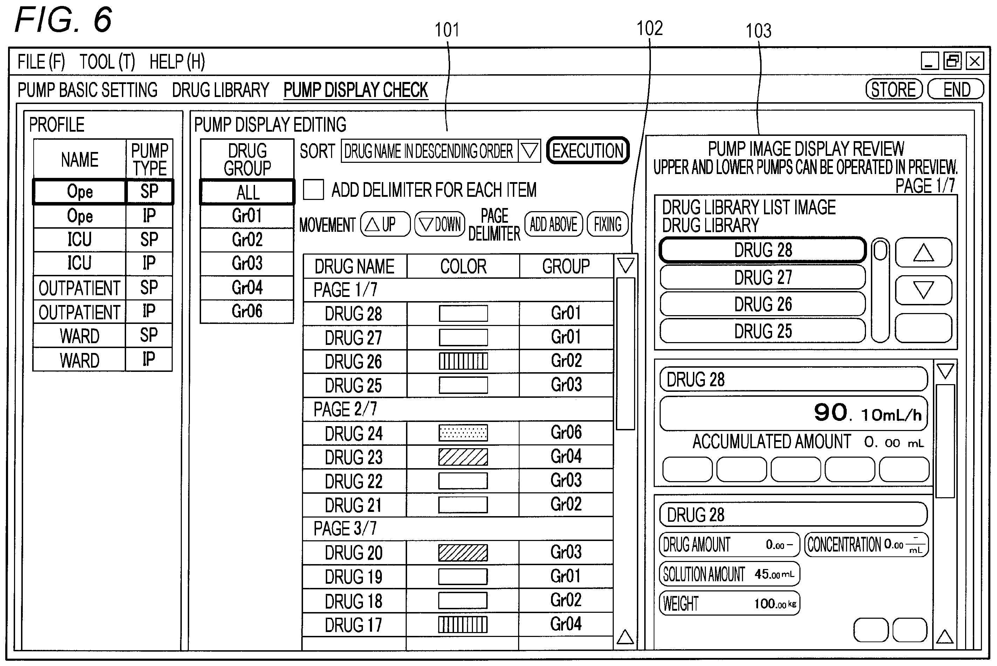

Fig. 6 is a diagram illustrating an example of a drug library editing screen in a case where the person in charge of editing work performs editing to sort drug names in descending order. The control unit 51 displays all drugs in the drug library in the form of a list in descending order of drug names on the page delimiter screen 102. When displayed in a list form, four drugs are displayed per page. Specifically, all the drugs in the drug library are delimited over 7 pages, the first page includes a drug 28, a drug 27, a drug 26, and a drug 25, the second page includes a drug 24, a drug 23, a drug 22, and a drug 21, and the third page includes a drug 20, a drug 19, a drug 18, and a drug 17. The same applies to the fourth and subsequent pages.

On the preview screen 103, four drugs 28, 27, 26, and 25 of the first page are displayed, and the second and subsequent pages can be similarly displayed by a scroll operation. When the person in charge of editing work selects the drug 28 among the four drugs on the first page, the control unit 51 can display administration information on the drug 28. In the example of Fig. 6, the flow rate (mL/h), the dose, and the like of the drug 28 are displayed. The preview screen 103 is an example and is not limited to the example of Fig. 6.

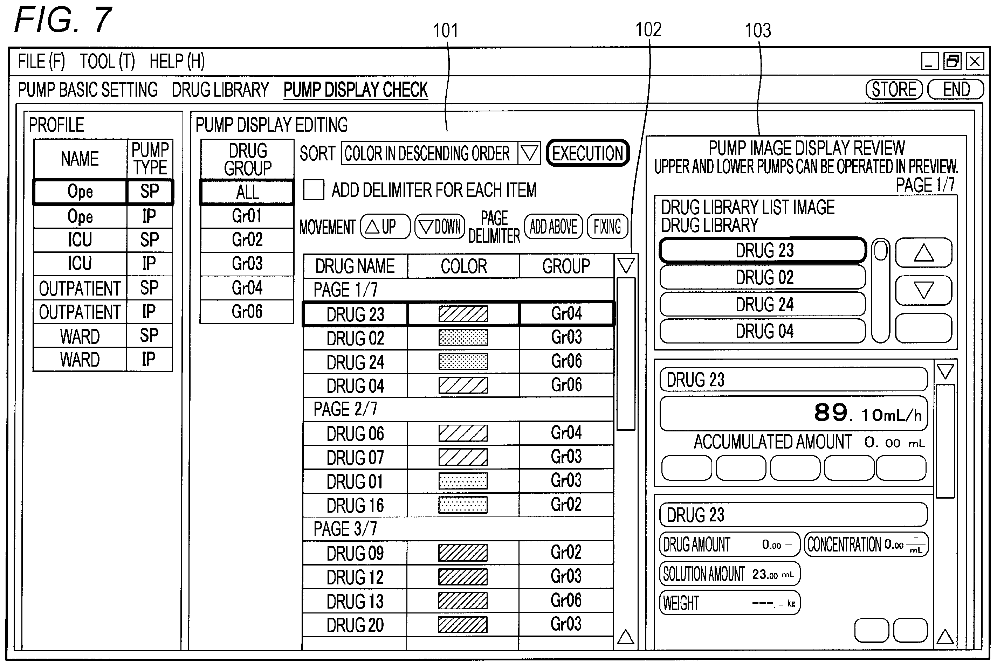

Fig. 7 is a diagram illustrating an example of a drug library editing screen in a case where the person in charge of editing work performs editing of rearranging colors in descending order. The control unit 51 displays all drugs in the drug library in the form of a list in descending order of colors on the page delimiter screen 102. When displayed in a list form, four drugs are displayed per page. Specifically, all the drugs in the drug library are sorted in descending order of colors over 7 pages, the first page includes the drug 23, the drug 02, the drug 24, and the drug 04, the second page includes the drug 06, the drug 07, the drug 01, and the drug 16, and the third page includes the drug 09, the drug 12, the drug 13, and the drug 20. The same applies to the fourth and subsequent pages.

On the preview screen 103, the four drugs 23, 02, 24, and 04 of the first page are displayed, and the second and subsequent pages can be similarly displayed by a scroll operation. When the person in charge of editing work selects the drug 23 among the four drugs on the first page, the control unit 51 can display administration information on the drug 23. In the example of Fig. 7, the flow rate (mL/h), the dose, and the like of the drug 23 are displayed. The preview screen 103 is an example and is not limited to the example of Fig. 7.

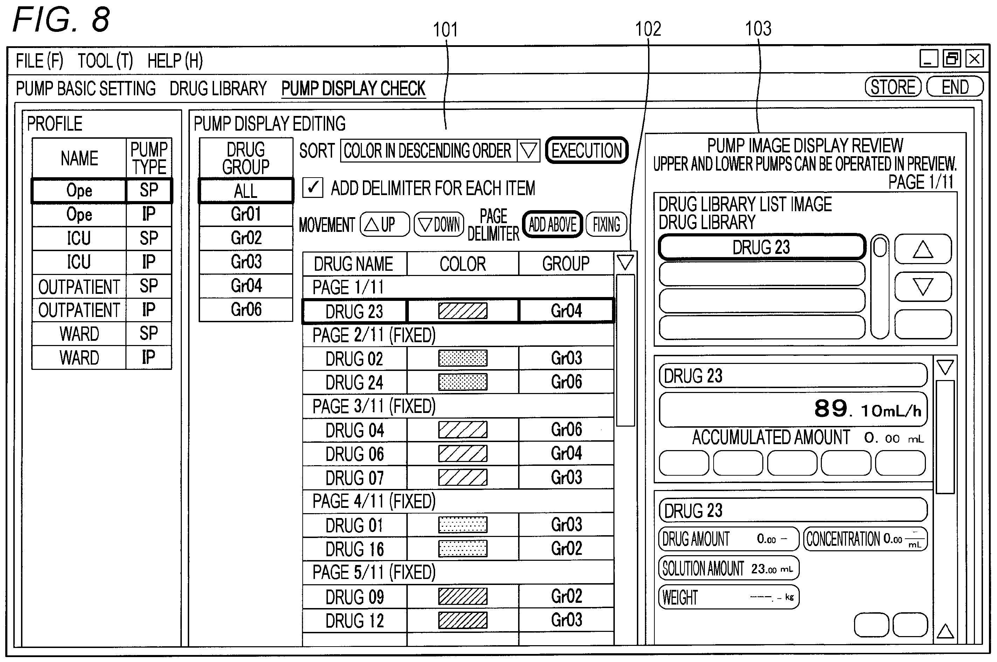

Fig. 8 is a diagram illustrating an example of a drug library editing screen in a case where the person in charge of editing work sorts colors in descending order and adds a delimiter for each item. The control unit 51 displays all drugs in the drug library in the form of a list in descending order of colors on the page delimiter screen 102. In Fig. 8, a page delimiter is added. Specifically, the first page includes only one drug 23, the second page includes two drugs 02 and 24, the third page includes three drugs 04, 06, and 07, and the fourth page includes two drugs 01 and 16. The same applies to the fifth and subsequent pages.

On the preview screen 103, the drug 23 of the first page is displayed, and the second and subsequent pages can be similarly displayed by a scroll operation. When the person in charge of editing work selects the drug 23 on the first page, the control unit 51 can display administration information on the drug 23. In the example of Fig. 8, the flow rate (mL/h), the dose, and the like of the drug 23 are displayed. The preview screen 103 is an example and is not limited to the example of Fig. 8.

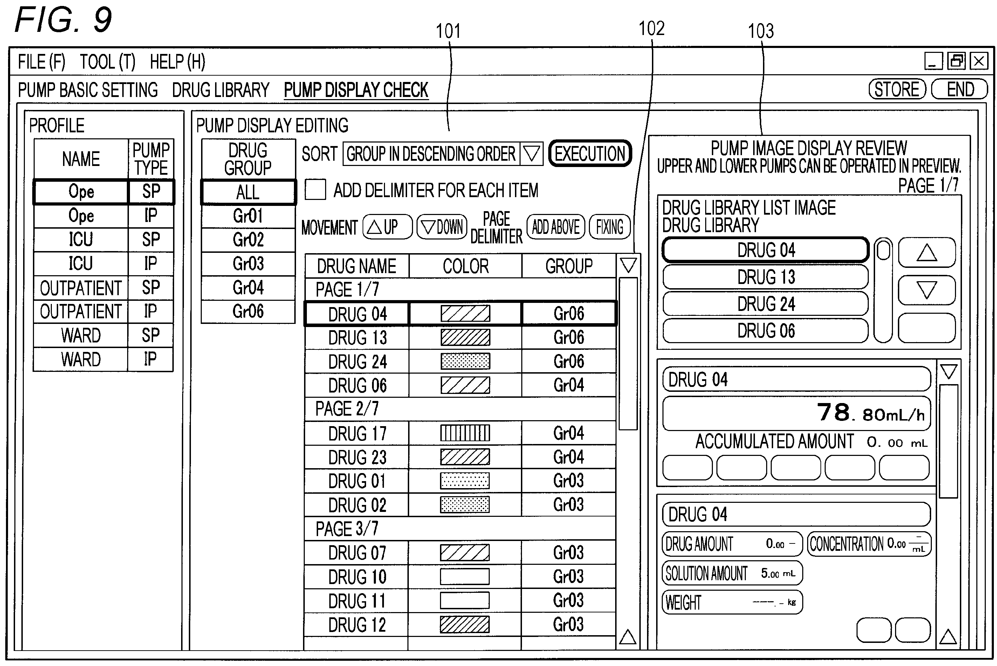

Fig. 9 is a diagram illustrating an example of a drug library editing screen in a case where the person in charge of editing work performs editing of rearranging groups in descending order. The control unit 51 displays all drugs in the drug library in the form of a list in descending order of groups on the page delimiter screen 102. When displayed in a list form, four drugs are displayed per page. Specifically, all the drugs in the drug library are sorted in descending order of colors over 7 pages, the first page includes the drug 04, the drug 13, the drug 24, and the drug 06, the second page includes the drug 17, the drug 23, the drug 01, and the drug 06, and the third page includes the drug 07, the drug 10, the drug 11, and the drug 12. The same applies to the fourth and subsequent pages.

On the preview screen 103, the four drugs 04, 13, 24, and 06 of the first page are displayed, and the second and subsequent pages can be similarly displayed by a scroll operation. When the person in charge of editing work selects the drug 04 among the four drugs on the first page, the control unit 51 can display administration information on the drug 04. In the example of Fig. 9, the flow rate (mL/h), the dose, and the like of the drug 04 are displayed. The preview screen 103 is an example and is not limited to the example of Fig. 9.

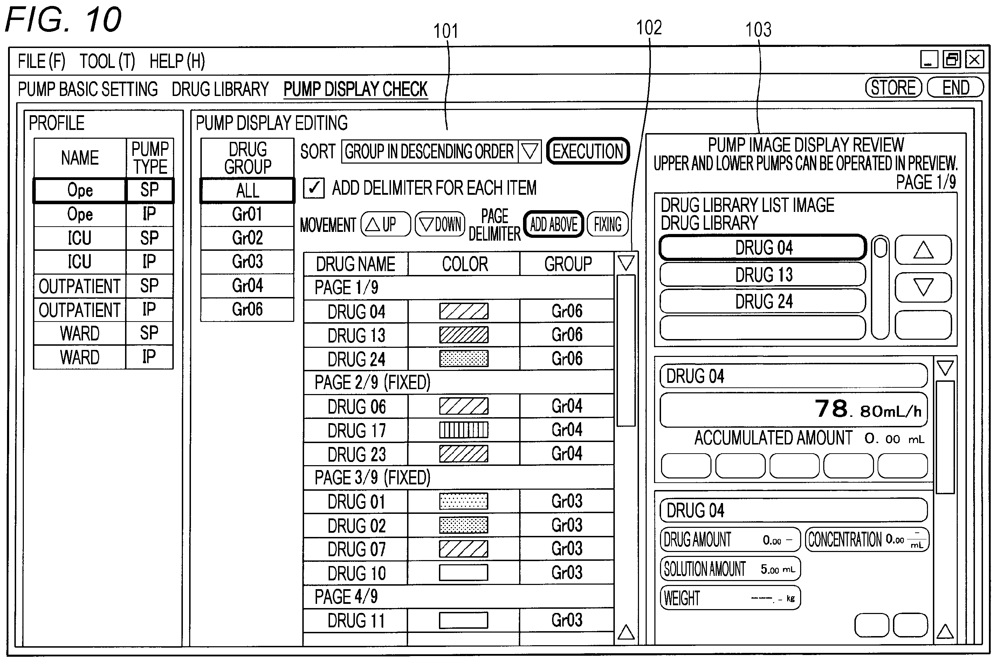

Fig. 10 is a diagram illustrating an example of a drug library editing screen in a case where the person in charge of editing work sorts groups in descending order and adds a delimiter for each item. The control unit 51 displays all drugs in the drug library in the form of a list in descending order of groups on the page delimiter screen 102. In Fig. 10, a page delimiter is added. Specifically, the first page includes three drugs 04, 13, and 24 included in the group Gr06, the second page includes three drugs 06, 17, and 23 included in the group Gr04, and the third page includes four drugs 01, 02, 07, and 10 included in the group Gr03. The same applies to the fourth and subsequent pages.

On the preview screen 103, three drugs 04, 13, and 24 of the first page are displayed, and the second and subsequent pages can be similarly displayed by a scroll operation. When the person in charge of editing work selects the drug 04 on the first page, the control unit 51 can display administration information on the drug 04. In the example of Fig. 10, the flow rate (mL/h), the dose, and the like of the drug 04 are displayed. The preview screen 103 is an example and is not limited to the example of Fig. 10.

As described above, the control unit 51 (computer program 57) acquires the drug library including the administration information for each drug, accepts the editing operation of the display format of the drug library displayed on the display unit 10 of the syringe pump 100 (medical pump) on the basis of the acquired drug library, and displays the preview information of the drug library displayed on the display unit 10 in the display format corresponding to the accepted editing operation.

As a result, a medical worker (person in charge of editing work) such as a pharmacist who edits the drug library can check the display and operation on the medical pump side at the time of editing, and can easily check the drug library displayed on the medical pump.

The control unit 51 may accept an editing operation related to a display order of drugs included in the drug library, and display the preview information of a list of drugs in a display order according to the accepted editing operation. As a result, when displaying the preview information of the drug library, the control unit 51 can display the display order of the drugs in a required order.

The control unit 51 may accept an editing operation related to the display order of at least one item of the drug name, the color (color code) of the drug syringe, and the drug group of the drug included in the drug library, and display the preview information of a list of drugs in the display order of the items according to the accepted editing operation. As a result, when displaying the preview information of the drug library, the control unit 51 can display the display order of the drug items (drug name, color code, or group) in a required order.

The control unit 51 may accept an editing operation of editing a page delimiter setting when displaying a list of drugs included in the drug library, and display the preview information of the list of drugs with page delimiter according to the accepted editing operation. More specifically, the control unit 51 may accept an editing operation of editing a page delimiter setting when a list of drugs is displayed in a display order of at least one item of a drug name, a color code, and a group of drugs included in the drug library, and display the preview information of the list of drugs by page delimiter according to the accepted editing operation. As a result, the control unit 51 can insert the page feed into the list of drugs displayed on the first page of the screen on the display unit of the medical pump, and grouping of drugs becomes possible, and it becomes easy to arrange drugs to be displayed on one page.

When displaying the preview information on the preview screen 103, in a case where the person in charge of editing work selects a drug included in the drug library displayed on the display unit of the medical pump, the control unit 51 may include administration information of the selected drug displayed on the display unit in the preview screen. As a result, the person in charge of editing work can also confirm the administration information of the drug at the time of editing the drug library.

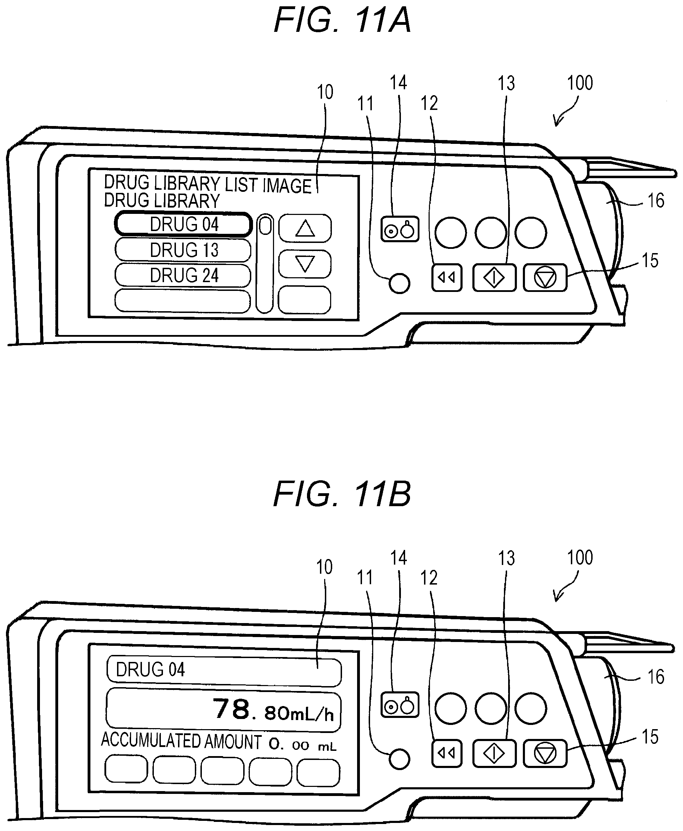

Figs. 11A and 11B are diagrams illustrating an example of a drug library displayed on the display unit 10 of the syringe pump 100. The non-volatile memory in the controller 20 of the syringe pump 100 stores a drug library edited by the drug library editing device 50. The controller 20 can display information of the stored drug library on the display unit 10.

In Fig. 11A, the drug on the first page of the drug library is displayed. Specifically, as illustrated in Fig. 10, three drugs 04, 13, and 24 included in the group Gr06 are displayed. When the medical worker using the medical pump performs a scroll operation, the drug on the second and subsequent pages can be displayed and checked. When the medical worker performs an operation of selecting the drug 04, as illustrated in Fig. 11B, the administration information of the drug 04 can be displayed and confirmed on the display unit 10. In Fig. 11B, when the medical worker performs a scroll operation, it is possible to display and confirm other administration information related to the drug 04, which is not displayed in Fig. 11B.

A medical worker such as a nurse who uses the medical pump at the medical site can attach the correct syringe 200 to the syringe pump 100 on the basis of the drug name and administration information of the drug displayed on the display unit 10 and set the flow rate and the dose according to the administration information. As a result, measures to prevent erroneous drug such as erroneous administration when a medical worker uses a medical pump are realized.

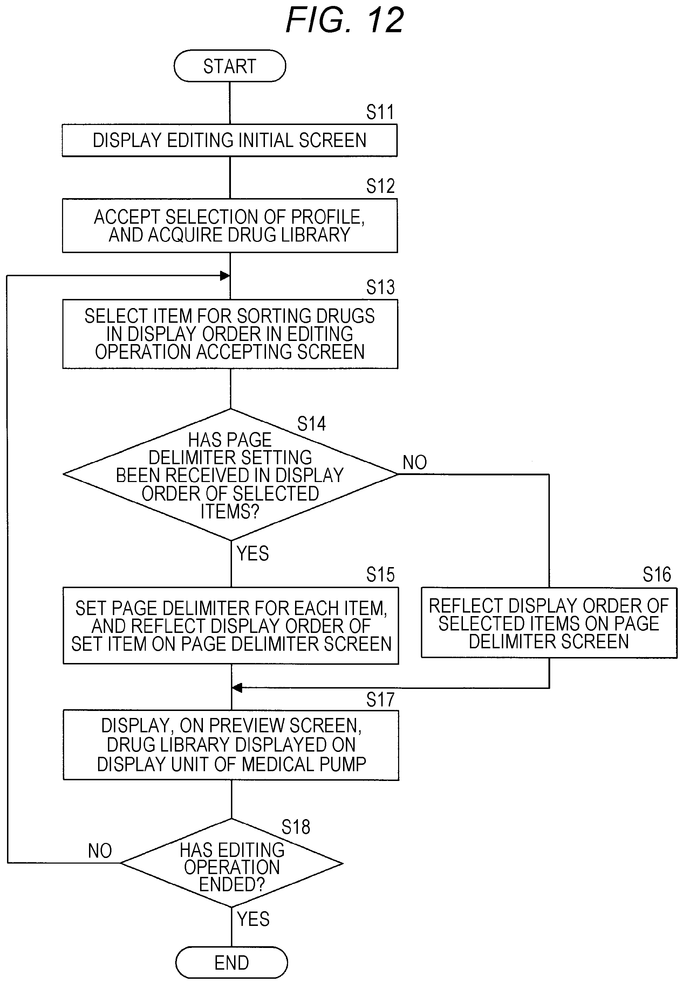

Fig. 12 is a diagram illustrating an example of a processing procedure by the drug library editing device 50. The control unit 51 displays an initial screen of a drug library editing screen (S11), accepts selection of a profile, and acquires a drug library corresponding to the accepted profile from the data server 60 (S12). The control unit 51 selects an item according to the selection operation of the item (drug name, color, group) for sorting the display order of the drug on the editing operation accepting screen 101 of the drug library editing screen (S13).

The control unit 51 determines whether the page delimiter setting has been received in the display order of the selected items (S14). In a case where the page delimiter setting has been received (YES in S14), the control unit 51 sets the page delimiter for each item, reflects the display order of the selected items on the page delimiter screen 102 (S15), and performs processing in step S17 described later.

In a case where the control unit 51 has not received the page delimiter setting (NO in S14), the control unit 51 reflects the display order of the selected items on the page delimiter screen 102 (S16), and displays the drug library displayed on the display unit 10 of the medical pump on the preview screen 103 (S17).

The control unit 51 determines whether the editing operation has ended (S18), and in a case where the editing operation has not ended (NO in S18), the processing from step S13 is continued. In a case where the editing operation has ended (YES in S18), the control unit 51 finishes the processing.

As described above, according to the present embodiment, the person in charge of editing work can confirm how the drug library is displayed on the display unit of the medical pump at the time of editing the drug library. In addition, the person in charge of editing work can group drugs displayed on one page by drug name, color code, or group of drugs. In addition, the person in charge of editing work can select the number of drugs to be displayed on one page in a range of one to a predetermined number (maximum display number), and can collect drugs to be displayed on one page for easy confirmation.

### Reference Signs List

- 1: communication network
- 50: drug library editing device
- 51: control unit
- 52: communication unit
- 53: memory
- 54: display panel
- 55: operation unit
- 56: storage unit
- 57: computer program
- 58: drug library
- 60: data server
- 61: drug library DB
- 100: syringe pump
- 10: display unit
- 200: syringe

## Claims

1. A computer program causing a computer to execute:
acquiring a drug library including administration information for each drug;
accepting an editing operation of a display format of the drug library displayed on a display unit of a medical pump based on the acquired drug library; and
displaying preview information of a drug library displayed on the display unit in a display format corresponding to the accepted editing operation.

2. The computer program according to claim 1, wherein
the editing operation includes a display order of drugs included in the drug library, and
the computer program causes a computer to execute displaying preview information of a list of drugs in a display order according to the accepted editing operation.

3. The computer program according to claim 1, wherein
the editing operation includes setting of page delimiter when displaying a list of drugs included in the drug library, and
the computer program causes a computer to execute displaying preview information of a list of drugs in page delimiter corresponding to the accepted editing operation.

4. The computer program according to any one of claims 1 to 3, wherein
the editing operation includes a display order of at least one item of a drug name, a color of a drug syringe, and a drug group, and
the computer program causes a computer to execute displaying preview information of a list of drugs in a display order of items according to the accepted editing operation.

5. The computer program according to claim 4, wherein
the editing operation includes setting of page delimiter when displaying a list of drugs in a display order of the items, and
the computer program causes a computer to execute displaying preview information of a list of drugs in page delimiter corresponding to the accepted editing operation.

6. The computer program according to any one of claims 1 to 3, wherein
the preview information includes administration information of a selected drug displayed on the display unit in a case where the drug included in the drug library displayed on the display unit is selected.

7. The computer program according to any one of claims 1 to 3, causing a computer to execute displaying:
an editing operation accepting screen that accepts the editing operation;
a page delimiter screen that includes page delimiter information of a drug library corresponding to the accepted editing operation; and
a preview screen that includes preview information of a drug library displayed on the display unit in a display format corresponding to the accepted editing operation.

8. A drug library editing device comprising:
a control unit, wherein
the control unit executes:
acquiring a drug library including administration information for each drug;
accepting an editing operation of a display format of the drug library displayed on a display unit of a medical pump based on the acquired drug library; and
displaying preview information of a drug library displayed on the display unit in a display format corresponding to the accepted editing operation.

9. A drug library editing system comprising:
a drug library editing device according to claim 8; and a medical pump in which a drug library edited by the drug library editing device is written.

10. A drug library editing method comprising:
acquiring a drug library including administration information for each drug;
accepting an editing operation of a display format of the drug library displayed on a display unit of a medical pump based on the acquired drug library; and
displaying preview information of a drug library displayed on the display unit in a display format corresponding to the accepted editing operation.
